Europäisches Patentamt

European Patent Office

Office européen des brevets

(11) Publication number: **0 068 465**
**B1**

# EUROPEAN PATENT SPECIFICATION

(45) Date of publication of patent specification: **21.11.85**

(21) Application number: **82105643.9**

(22) Date of filing: **25.06.82**

(51) Int. Cl.⁴: **A 61 K 39/42,** A 61 K 39/44, G 01 N 33/576

(54) Non-A, non-B hepatitis-associated antibody conjugate, the use thereof and detection reagent.

(30) Priority: **25.06.81 JP 97425/81**

(43) Date of publication of application:
**05.01.83 Bulletin 83/01**

(45) Publication of the grant of the patent:
**21.11.85 Bulletin 85/47**

(84) Designated Contracting States:
**BE CH DE FR GB IT LI NL SE**

(56) References cited:
**WO-A-80/02598**
**WO-A-82/03330**

**BIOLOGICAL ABSTRACTS, vol. 69, 1980, ref. 58712, L. VITVITSKI et al.: "Detection of virus-associated antigen in serum and liver of patients with non-.A non-B hepatitis"**

(73) Proprietor: **Eisai Co., Ltd.**
**6-10, Koishikawa 4-chome Bunkyo-ku Tokyo 112 (JP)**

(72) Inventor: **Tomatsu, Junichi**
**Warabi residence 511 4-1-5, Kitamachi Warabi-shi Saitama (JP)**
Inventor: **Morimoto, Tomiaki**
**2-10-10, Fuseshin-machi Kashiwa-shi Chiba (JP)**
Inventor: **Shikata, Toshio**
**1-17-2, Motokitakata Ichikawa-shi Chiba (JP)**

(74) Representative: **Hansen, Bernd, Dr.rer.nat. et al**
**Hoffmann, Eitle & Partner Patentanwälte Arabellastrasse 4**
**D-8000 München 81 (DE)**

Courier Press, Leamington Spa, England.

## Description

This invention relates to a type non-A, non-B hepatitis-associated antibody conjugate, the use thereof and a detection reagent containing the same.

The existance of viral hepatitis of types A and B is known and antigen-antibody systems associated with these types of hepatitis have also been clarified. Immunoserological ˙ diagnosis using such systems has become possible and development and introduction of vaccines effective against types A and B hepatitis have been accomplished.

In the course of research, however, the existance of viral heptatis which belongs neither to hepatitis type A nor to hepatitis type B has become known. The frequency of occurrence thereof is higher than expected. Non-A, non-B hepatitis accounts for more than 80 up to 90% of post-transfusion hepatitis and it has been reported that non-A, non-B hepatitis also accounts for about 50% of sporadic acute hepatits. It has been clarified that even among normal blood donors, a considerable number of donors carry non-A, non-B hepatitis virus(es). The following literature references set forth the state of the art relating to non-A, non-B hepatitis:

1) Prince, A. M. et al.: Long-incubation post-transfusion hepatitis without serological evidence of exposure to hepatitis-B virus. Lancet. II: 241—246, 1974.
2) Alter, H. J. et al.: Clinical and serological analysis of transfusion-associated hepatitis. Lancet. II: 838—841, 1975.
3) Feinstone, S. M. et al.: Transfusion-associated hepatitis not due to viral hepatitis type A or B. N. Engl. J. Med. *292*: 767—770, 1975.
4) Meyers, J. D. et al.: Parenterally transmitted non-A, non-B hepatitis. Ann. Intern. Med. *87*: 57—59, 1977.
5) Villarejos, V. M. et al.: Evidence for viral hepatitis other than type A or type B among persons in Costa Rica. N. Engl. J. Med. *293:* 1350—1352, 1975.
6) Dienstag, J. L. et al.: Etiology of sporadic hepatitis B surface antigen-negative hepatitis. Ann. Intern. Med. *87*: 1—6, 1977.

The term "non-A, non-B hepatitis" used herein refers to those kinds of hepatitis which are believed to occur because of virus(es) other than the heretofore known viruses, such as type A hepatitis virus, type B hepatitis virus, cytomegalovirus, Epstein-Barr virus and the like, which non-A, non-B hepatitis virus(es) participate in the disease as pathogens and do not provoke a significant rise of HAV antibody, HBs antibody, HBc antibody and HBe antibody. Accordingly non-A, non-B hepatitis patients can be defined as those who suffer from hepatitis resulting from hepatitis virus(es), but who are negative in both the type A and type B hepatitis diagnoses, and are therefore excluded from having either hepatitis type A or hepatitis type B. Generally, in comparison with hepatitis type B, the average incubation period from transfusion until outbreak for non-A, non-B hepatitis is shorter, and the maximal value of GPT is also lower. However, non-A, non-B hepatitis has a longer duration so that the GPT abnormality, for example, becomes chronic and lasts longer. In any case, non-A, non-B hepatitis accounts for the major proportion of post-transfusion hepatitis, and liver diseases caused by the non-A, non-B hepatitis virus(es) include a variety of diseases such as liver-cirrhosis, liver cancer, and the like. Accordingly, proper counter-measures must be devised as soon as possible. In particular, means for accurately detecting the non-A, non-B hepatitis-associated antigen in transfusion blood must be developed as soon as possible. As to non-A, non-B hepatitis, however, the virus(es) that cause the disease have not yet been clarifed essentially so that an antigen-antibody system associated therewith has not yet been established. Hence, proper diagnosis and vaccine production have not been achieved to date.

In view of the background situation described above, the inventors of the present invention first attempted to isolate, as a separate substance, an associated antigen which is a general factor in and is specific to non-A, non-B hepatitis. The present inventor succeeded in obtaining the contemplated substance by isolation and purification from a non-A, non-B hepatitis autopsy liver. The present inventor further attempted to prepare a non-A, non-B hepatitis-associated antibody and antibody conjugate from the substance by an immunological method and succeeded, thereby completing the present invention.

As is obvious from the above explanation, an object of the present invention is to provide an associated antibody conjugate which is specific to non-A, non-B hepatitis and also to make it possible to detect a non-A, non-B hepatitis-associated antigen by using such an antibody conjugate.

The present invention concerns a conjugate comprising (a) an antibody associated with non-A, non-B hepatitis which antibody exhibits a specific antigen-antibody reaction with an antigen associated with non-A, non-B hepatitis, said antigen being obtained by isolation and purification from the liver of a mammal infected with non-A, non-B hepatitis, said antigen having the following physicochemical properties:

molecular weight: 1,500,000 or higher, as determined by gel filtration.

sedimentation constant $(10^{-13})$: 51.5S, as determined by ultracentrifugal analysis;

buoyant density $(g/cm^3)$: 1.15—1.25 in cesium chloride and in potassium bromide;

particle diameter: approximately 26—37 nm; and

electrophoretic mobility: $\alpha_2$—$\alpha_1$ globulin region, in agarose gel; and

(2) a substance selected from fine carrier particles suitable for passive hemagglutination method, labelling isotopes suitable for radioimmunoassay, and enzymes suitable for enzyme-linked immunoadsorbent assay.

The invention also comprises a detection reagent which contains as its principal ingredient the above-identified antibody conjugate.

The present invention will now be described in detail.

The non-A, non-B hepatitis-associated antibody exhibits a specific antigen-antibody reaction with the non-A, non-B hepatitis-associated antigen. The non-A, non-B hepatitis-associated antigen is obtained by isolation and purification from an autopsy liver of a non-A, non-B hepatitis patient and is characterized by the following physicochemical properties. The molecular weight of this antigen is at least 1,500,000 when determined by gel filtration. Its sedimentation constant ($10^{-13}$) is 51.5S when measured with the Beckmann Model E instrument. Its buoyant density ($g/cm^3$) in cesium chloride or in potassium bromide solution is 1.15 to 1.25 when determined with an Abbe's refractometer. The particle size of the antigen is from 26 to 37 nm under electron microscope observation (average 31.5 nm). The electrophoretic mobility of the antigen is in the $\alpha_2$—$\alpha_1$ globulin range when determined by immunoelectrophoresis.

The antigen-antibody reaction which the antibody exhibits with the non-A, non-B hepatitis-associated antigen described above is specific, as will be demonstrated in Experimental Example 1 below. Consequently, the antibody can be defined in terms of its ability to undergo this antigen-antibody reaction. This reaction is also quantitative, as will be illustrated in Experimental Example 2. Accordingly, non-A, non-B hepatitis-associated antigen levels can be detected by means of this reaction.

The above antibody can be directly obtained from the serum of a patient who has had frequent transfusions or the convalescent phase serum from non-A, non-B hepatitis patient, and used as the starting material by isolating and purifying the serum. Generally, however, the antibody is indirectly obtained by an immunological method using animals other than man. For example, as will be shown in Example 1, a rabbit is repeatedly inoculated, for immunization, with the non-A, non-B hepatitis-associated antigen, together with Freund's complete adjuvant, and the resulting antiserum is subjected to IgG purification fractionation, for example, to obtain the respective antibody.

The non-A, non-B hepatitis-associated antigen to be used can be obtained from the autopsy liver from a patient infected with non-A, non-B hepatitis, as the starting material, by a suitable combination of ordinary protein fractionation methods, such as density gradient centrifugation, salting out, electrophoresis, gel filtration, affinity chromatography, isoelectrofocusing, ultrafiltration, Cohn's fractionation, and so forth. For instance, the autopsy liver supernatant homogenate is first subjected to column chromatography using Sephacryl S-200® and the resulting antigen-positive fraction is concentrated with ultrafiltration. In the following ultracentrifugation and dialysis are carried out. It is preferred to carry out ultracentrifugation once or several times by using successively both sucrose density gradient centrifugation and cesium chloride density gradient centrifugation.

The invention also provides a detection reagent useful in a method such as reverse passive hemagglutination (R-PHA), radioimmunoassy (RIA), or enzyme-linked immunoadsorbent assay (ELISA). For these detection methods, it is necessary that the antibody is prepared in the form of a suitable conjugate. For example, when reverse passive hemagglutination (R-PHA) is employed, the antibody of the present invention must be coupled with fine particles. Although it is preferred to use the erythrocytes of mammals or birds, it is also possible to use fine particles of about 1 to about 10 μ, such as fine particles of polystyrene latex, polyester latex, vinyl chloride, bentonite, glass beads, and so forth. In order to bind the antibody of the present invention with these fine particles, it is possible to use, for example, glutaraldehyde, formaldehyde, tannic acid, bisdiazotized benzidine, chromium chloride, or carbodiimide.

When the radioimmunoassay (RIA) test is used, the antibody of the present invention must be labelled with an isotope. $^{125}$I or $^{131}$I can be used as the isotope and can be combined with the antibody of the invention by the chloramine T method.

When the enzyme-linked immunoadsorbent assay (ELISA) method is used, the antibody of the present invention must be combined with an enzyme. Examples of usable enzymes include glucose oxidase, alkali phosphatase, peroxidase and β-galactosidase. Glutaraldehyde can be used as the coupling agent.

The figure is a schematic diagram showing the state of formation of precipitin lines according to the micro-Ouchterlony's test as described in Experimental Example 1.

The usefulness of the above-described antibody, the antibody conjugate and the detection reagent containing said antibody conjugate will be shown with reference to the following illustrative Experimental Examples.

Experimental Example 1
Samples:

a. Non-A, non-B hepatitis-associated antigen obtained by isolation and purification from a non-A, non-B hepatitis autopsy liver and having the aforementioned physicochemical properties (also used in Experimental Example 2)

b. Convalescent phase serum from non-A, non-B hepatitis patient

c. Supernatant homogenate of a non-A, non-B hepatitis autopsy liver

d. Supernatant homogenate of normal human liver

e. Normal human serum

f. Antibodies of the present invention obtained in Example 1 (obtained by subjecting immune antiserum of a test rabbit to IgG purification fractionation)

Method:

0.8% (W/V) of agarose A-45® (Nakarai Kagaku), 0.02M of EDTA·3Na, 0.1M of sodium chloride and 0.1% of $NaN_3$ were dissolved in a Tris-HCl buffer (0.01M, pH 7.5) and a 1.5 mm-thick gel plate was prepared. The amounts given hereafter are (W/V), weight in volume concentration, unless noted otherwise. A detection test was carried out for each sample in accordance with the micro-Ouchterlony's test.

Results:

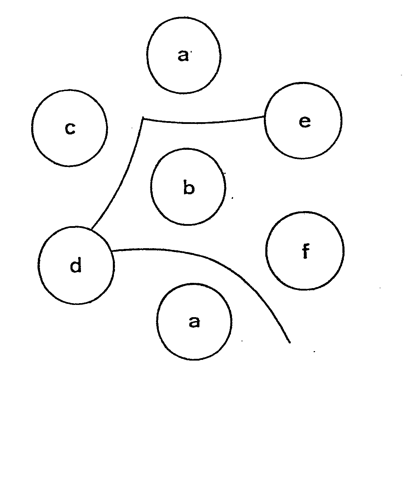

The results are shown in the attached drawing. Round zones a through f in the drawing represent the disposition areas in which the corresponding samples were spotted. In Figure 1, the precipitin line between b-a completely fuses with the line between b-c, and the precipitin line between a-b completely fuses with the line between a—f. In other words, a and c had immunologically the same antigen property with respect to b, and b and f had immunologically the same antibody specificity with respect to a. On the other hand, the normal human liver homogenate d and the normal human serum e did not show the cross reaction at all. As described above, the non-A, non-B hepatitis autopsy liver represents the liver of a human patient having a type of hepatitis originating from hepatitis virus(es), but who is negative to both hepatitis type A diagnosis and hepatitis type B diagnosis, and who, therefore, is excluded from being infected with hepatitis types A and B. Accordingly, the antibody f of the present invention produced immunologically by use of the antigen from the non-A, non-B hepatitis autopsy liver, as in Example 1, showed a specific antigen-antibody reaction with the non-A, non-B hepatitis-associated antigen a. It was thus found that the antibody could also be obtained from the serum of a patient convalescing from non-A, non-B hepatitis.

Experimental Example 2

Samples:
  a.  Non-A, non-B hepatitis-associated antigen
  b.  Liver homogenate supernatant from non-A, non-B hepatitis patient
  c.  Normal human liver homogenate supernatant.

Four kinds of antibody-sensitized sheep erythrocytes were prepared by following the same procedures as described in Example 2 except that solutions were used which were prepared by diluting the purified IgG solution with a phosphate buffered saline solution so that the antibody dilution ratios became 1:10, 1:20, 1:40 and 1:80, respectively.

Method:

25 µl of a phosphate buffered saline (0.15 M, pH 7.2, PBS) containing 2% normal rabbit serum were dropped into micro-titration plates (V-type) and each sample was diluted with said PBS by $2^n$ times in two series (serial 2-fold dilution). 25 µl of a phosphate buffered saline (0.15 M, pH 7.2) containing 2% normal rabbit blood serum was added to the samples of one series while 25 µl of the serum from non-A, non-B hepatitis patient containing the antibody of the present invention was added to the samples of the other series. After incubation at 37°C for 30 minutes, antibody-sensitized sheep erythrocytes were added to each series. Then the plates were each subjected to further incubation at room temperature for 2 hours to confirm occurrence of agglutination. Samples were determined as positive whose agglutination value was 3 tubes or more (dilution of 8 times or more) on the buffer side and whose agglutination inhibition was confirmed for at least 2 tubes. The antigen titer was expressed as the highest dilution of the sample that showed hemagglutination.

Results:

The results are shown in Table 1.

Table 1 antigen titer

| Antibody dilution ratio | Sample a | Sample b | c |
|:---:|:---:|:---:|:---:|
| 1:10 | 2,000 | 1,000 | — |
| 1:20 | 2,000 | 1,000 | — |
| 1:40 | 2,000 | 1,000 | — |
| 1:80 | 500 | 200 | — |

In this table, the symbol "-" represents an agglutination value of two or below.

It can be seen from Table 1 that the sensitivity of the agglutination reaction of the sheep erythrocytes sensitized by the antibody of the present invention was 50 to 100 times that of the MO method and was extremely high. It can also be concluded that the specificity of an agglutinogen could be clearly confirmed from the existence of agglutination inhibition due to the serum from non-A, non-B hepatitis patient containing the antibody of the present invention.

The present invention will be described in further detail with reference to the following examples of preparation of the antibody of the invention.

Example 1

Freund's complete adjuvant was added in a ratio of 1:1 to a solution containing non-A, non-B hepatitis-associated antigen obtained by isolation and purification from non-A, non-B hepatitis autopsy liver and having the aforementioned physicochemical properties, and an emulsion was formed. 0.5 ml portions of the emulsion were injected into the back of a rabbit, subcutaneously and intracutaneously, and into the food pad of the rabbit. One week later, the equal volume of the emulsion was additionally injected for immunization. One month after the first immunization, a double quantity of the emulsion was further injected. One week after the last immunization, the blood of the rabbit was collected several times in order to separate the serum therefrom. The equal volume of a normal human liver homogenate supernatant was added to this anti-serum, and the resulting mixture was left standing at 37°C for one hour, then at 4°C over night. The mixture was centrifuged at 9,000 g for 10 minutes to collect a supernatant fraction containing the antibody of the invention. The antibody of the present invention was then stored in the form of an aqueous solution and was used in Example 2 below.

Next, 50 ml of the antibody of the present invention was dialyzed at 4°C for one day and one night using a 0.01M phosphate buffer (pH 8.0) as an external solution. Thereafter, DEAE cellulose equilibrated sufficiently with a 0.01M phosphate buffer (pH 8.0) was packed in a chromatography column and the antibody of the present invention was added to the column, so that serum proteins other the γ-globulin would be adsorbed. Subsequently, the unadsorbed γ-globulin was eluted, recovered by the phosphate buffer (0.01M, pH 8.0), and subjected to ultra-filtration using a PM-10 membrane to adjust the protein concentration to about 1.0 mg/ml. The resulting purified IgG material of the antibody of the present invention was used in Examples 2, 3 and 4 below.

Example 2
Sheep blood was placed in a centrifugal tube and centrifuged at 1,000 g for 10 minutes using a saline solution. Centrifugation was repeated five times to wash the erythrocytes. A phosphate buffered saline solution of pH 7.5 and 0.15 M was added to this erythrocyte suspension in a 5% concentration. A glutaraldehyde solution prepared in a 2.5 % concentration, using the same phosphate buffered saline solution, was added to the erythrocyte suspension, at a volume ratio of said glutaraldehyde to said erythrocyte suspension of 1:5. Then the mixed solution was reacted at room temperature for about 5 hours with stirring to fix the erythrocytes. The solution was then centrifuged to obtain the fixed erythrocytes, which were washed several times by centrifugation using a saline solution.

The fixed erythrocytes were prepared in a 5 % suspension using the 0.15 M phosphate buffered saline solution of pH 7.5. A tannic acid solution adjusted to 5 mg/dl using the same phosphate buffered saline solution was added in an equivalent quantity and the mixture was stirred for 30 minutes. The mixture was centrifuged to obtain the tanned and fixed erythrocytes, which were then washed several times by centrifugation using a saline solution. The phosphate buffered saline solution was added to the resulting tanned and fixed erythrocytes to form a 5% erythrocyte suspension.

This erythrocyte suspension was mixed with the same quantity of a solution obtained by diluting the IgG purified material of Example 1 in a protein concentration of about 50 µg/ml (antibody dilution ratio 1:20) with the phosphate buffered saline solution, and the mixed solution was stirred at room temperature for 60 minutes to sensitize the erythrocytes. The solution was centrifuged to obtain the sensitized erythrocytes, which were washed several times by centrifugation using a saline solution. A phosphate buffered saline solution containing 2% normal rabbit blood serum was added to the resulting sensitized erythrocytes to form a 7% suspension. The sensitized erythrocytes were used as a detection reagent according to the R-PHA process.

Example 3
The IgG purified material obtained in Example 1 was dissolved in a 0.05M phosphate buffered saline solution of pH 7.5 so as to adjust the protein concentration to 1 mg/ml. 50 µl of one mCi $^{125}$I-Na was added to 10 µl of this solution, and 10 µl of a solution obtained by dissolving chloramine T in the abovementioned phosphate buffered saline solution at a concentration of 1.5 mg/ml was further added. The mixed solution was stirred for 30 seconds, and 100 µl of a solution obtained by dissolving sodium metabisulfite in the abovementioned phosphate buffered saline solution at a ratio of 2 mg/ml was added to the solution to stop the reaction. 100 µl of a solution obtained by dissolving potassium iodide in the abovementioned phosphate buffered saline at a concentration of 10 mg/ml was added to this reaction solution and an $^{125}$I-labelled substance was immediately separated from $^{125}$I by gel filtration with Sephadex G-50®. The specific activity of the resulting $^{125}$I-labelled substance was about 5 to 20 µCi/µg. This $^{125}$I-labelled substance was used as a detection reagent according to the RIA process.

Example 4
The IgG purified material obtained in Example 1 was concentrated to 5 to 10 mg/ml (in a 0.05M phosphate buffered saline solution) and alkali phosphatase (5 mg/ml) was added in the proportion of 0.3 ml to 0.1—0.2 ml of the concentrate. Next, 50 µl of 2.5% glutaraldehyde was added and the mixture was stirred at room temperature for 30 minutes. The solution was dialyzed over night with a 0.05M Tris-HCl buffer (pH 8.0) and then centrifuged at 1,000 g for 10 minutes. The resulting supernatant substance was used as a detection reagent according to the EIA using the enzyme-linked antibody.

With particular reference to processes for obtaining the non-A, non-B hepatitis-associated antigen associated with the antibody of the present invention, reference is made to EP—A—

0066 296 entitled *Non-A, Non-B Hepatitis-Associated Antigen and Diagnostic Reagent*.

**Claims**

1. A conjugate comprising (1) an antibody associated with non-A, non-B hepatitis which antibody exhibits a specific antigen-antibody reaction with an antigen associated with non-A, non-B hepatitis, said antigen being obtained by isolation and purification from the liver of a mammal infected with non-A, non-B hepatitis, said antigen having the following physicochemical properties:

molecular weight: 1,500,000 or higher, as determined by gel filtration;

sedimentation constant ($10^{-13}$): 51.5S, as determined by ultracentrifugal analysis;

buoyant density ($g/cm^3$): 1.15—1.25 in cesium chloride and in postassium bromide;

particle diameter: approximately 26—37 nm; and

electrophoretic mobility: $\alpha_2$—$\alpha_1$ globulin region, in agarose gel; and

(2) a substance selceted from fine carrier particles suitable for passive hemagglutination method, labelling isotopes suitable for radioimmunoassay, and enzymes suitable for enzymelinked immunoadsorbent assay.

2. A conjugate as claimed in Claim 1, wherein said fine particles are sheep erythrocytes, said isotope is $^{125}$I or $^{131}$I and said enzyme is alkali phosphatase.

3. A detection reagent adapted to detect an antigen associated with non-A, non-B hepatitis containing, as its principal ingredient, a conjugate of an antibody associated with non-A, non-B hepatitis which antibody exhibits a specific antigen-antibody reaction with an antigen associated with non-A, non-B hepatitis, said antigen being obtained by isolation and purification from the liver of a mammal infected with non-A, non-B hepatitis, said antigen having the following physicochemical properties:

molecular weight: 1,500,000 or higher, as determined by gel filtration;

sedimentation constant ($10^{-13}$): 51.5S, as determined by ultracentrifugal analysis;

buoyant density ($g/cm^3$): 1.15—1.25, in cesium chloride and in potassium bromide;

particle diameter: approximately 26—37 nm; and

electrophoretic mobility: $\alpha_2$—$\alpha_1$ globulin region, in agarose gel;

and a substance selected from fine carrier particles suitable for passive hemagglutination method, labelling isotopes suitable for radioimmunoassay and enzymes suitable for enzymelinked immunoadsorbent assay.

4. A detection reagent as claimed in Claim 3, wherein said fine particles are sheep erythrocytes, said isotope is $^{125}$I or $^{131}$I and said enzyme is alkali phosphatase.

5. A detection reagent according to Claim 3, wherein said antibody is obtained by a process comprising the steps of inoculating a warmblooded animal with said antigen, thereby causing formation of an antiserum; and removing said antiserum from said animal and subjecting said antiserum to IgG purification fractionation to obtain said antibody.

6. A detection reagent as claimed in claim 5, wherein said antibody is obtained from a rabbit.

7. A method for detecting an antigen associated with non-A, non-B hepatitis with the use of a detection agent according to claims 3 to 6.

**Patentansprüche**

1. Konjugat, welches (1) einen mit Nicht-A, Nicht-B-Hepatitis in Beziehung stehenden Antikörper, wobei der Antikörper eine spezifische Antigen-Antikörper-Reaktion mit einem mit Nicht-A, Nicht-B-Hepatitis in Beziehung stehenden Antigen ergibt, und das Antigen erhalten wird durch Isolierung und Reinigung aus der Leber eines Säugers, welche mit Nicht-A, Nicht-B-Hepatitis infiziert ist, wobei das Antigen die folgenden physikochemischen Eigenschaften aufweist:

Molekulargewicht: 1.500.000 oder darüber, bestimmt durch Gelfiltration;

Sedimentationskonstante ($10^{-13}$): 51,5S, bestimmt durch analytische Ultrazentrifugation;

Auftriebsdichte ($g/cm^3$): 1,15 bis 1,25 in Cäsiumchlorid und in Kaliumbromid;

Teilchendurchmesser: ca. 26 bis 37 nm; und

elektrophoretische Beweglichkeit: $\alpha_2$—$\alpha_1$-Globulinbereich, in Agarosegel; und

(2) eine Substanz ausgewählt aus feinen Trägerteilchen, die für die passive Hämagglutinationsmethode geeignet sind, Markerisotopen, die zur Radioimmun-Bestimmung geeignet sind, und Enzyme, die zur Enzym-Immunoadsorbens-Bestimmung geeignet sind, umfasst.

2. Konjugat nach Anspruch 1, dadurch gekennzeichnet, dass die feinen Teilchen Schaf-Erythrocyten, das genannte Isotop $^{125}$I oder $^{131}$I und das genannte Enzym alkalische Phosphatase darstellen.

3. Nachweisreagenz zum Nachweis eines mit Nicht-A, Nicht-B-Hepatitis in Beziehung stehendem Antigen, welches als Hauptbestandteil ein Konjugat aus einem mit Nicht-A, Nicht-B-Hepatitis in Beziehung stehenden Antikörper, wobei der Antikörper eine spezifische Antigen-Antikörper-Reaktion mit einem mit Nicht-A, Nicht-B-Hepatitis in Beziehung stehendem Antigen ergibt, und das genannte Antigen erhalten wird durch Isolierung und Reinigung aus der Leber eines Säugers, welche mit Nicht-A, Nicht-B-Hepatitis infiziert ist, wobei das Antigen die folgende physikochemischen Eigenschaften aufweist:

Molekulargewicht: 1.500.000 der darüber, bestimmt durch Gelfiltration;

Sedimentationskonstante ($10^{-13}$): 51,5S, bestimmt durch analytische Ultrazentrifugation;

Auftriebsdichte ($g/cm^3$): 1,15 bis 1,25 in Cäsiumchlorid und in Kaliumbromid;

Teilchendurchmesser: ca. 26 bis 37 nm; und elektrophoretische Beweglichkeit: $\alpha_2$—$\alpha_1$-Globulinbereich, in Agarosegel;

und eine Substanz, ausgewählt aus feinen Trägerteilchen, die für die passive Hämagglutinations-Methode geeignet sind, Markerisotope, die für die Radioummunbestimmung geeignet sind, und Enzyme, die zur Enzym-Immunoadsorbensbestimmung geeignet sind, umfasst.

4. Nachweisreagenz nach Anspruch 3, dadurch gekennzeichnet, dass die genannten feinen Partikel Schaf-Erythrocyten, das genannte Isotop $^{125}$I oder $^{131}$I und das genannte Enzym alkalische Phosphatase darstellen.

5. Nachweisreagenz nach Anspruch 3, wobei der genannte Antikörper durch ein Verfahren erhalten wird, das durch die folgenden Schritte gekennzeichnet ist: Inokulieren eines warmblütigen Tieres mit dem genannten Antigen, wodurch es zur Bildung von Antiserum kommt; und Entnehmen des genannten Antiserums aus dem genannten Tier, und IgG-Reinigungsfraktionierung des genannten Antiserums unter Erhalt des genannten Antikörpers.

6. Nachweisreagenz nach Anspruch 5, dadurch gekennzeichnet, dass der genannte Antikörper aus einem Kaninchen erhalte wird.

7. Verfahren zum Nachweis eines mit Nicht-A, Nicht-B-Hepatitis in Beziehung stehenden Antigens unter Verwendung eines Nachweisreagenz gemäss den Ansprüchen 3 bis 6.

**Revendications**

1. Produit conjugué comprenant (1) un anticorps associé à l'hépatite non-A, non-B, lequel anticorps montre une réaction antigène-anticorps spécifique avec un antigène associé à l'hépatite non-A, non-B, l'antigène étant obtenu par isolement et purification à partir du foie d'un mammifère atteint d'une hépatite non-A, non-B, ledit antigène ayant les propriétés physicochimiques suivantes:

mass moléculaire: 1 500 000 ou plus, déterminée par filtration sur gel;

constante de sédimentation $(10^{-13})$: 51,5 S, déterminée par analyse par ultracentrifugation;

masse volumique apparente $(g/cm^3)$: 1,15—1,25 dans le chlorure de césium et dans le bromure de potassium;

diamètre des particules: environ 26 à 37 nm; et mobilité électrophorétique: gamme des $\alpha_2$—$\alpha_1$ globulines, dans un gel d'agarose; et

(2) une substance choisie parmi de fines particules de support appropriées pour une méthode d'hémagglutination passive, des isotopes de marquage appropriés pour un dosage radioimmunologique et des enzymes convenant à un dosage d'immunoadsorbant fixé sur une enzyme.

2. Produit conjugué selon la revendication 1, dans lequel les fines particules sont des érythrocytes de mouton, l'isotope est $^{125}$I ou $^{131}$I et l'enzyme est la phosphatase alcaline.

3. Réactif de détection permettant de détecter un antigène associé à l'hépatite non-A, non-B, contenant en tant que principal ingrédient, un produit conjugué d'anticorps associé à l'hépatite non-A, non-B, lequel anticorps montre une réaction antigène-anticorps spécifique avec un antigène associé à l'hépatite non-A, non-B, l'antigène étant obtenu par isolement et purification à partir du foie d'un mammifère atteint d'une hépatite non-A, non-B, ledit antigène ayant les propriètés physicochimiques suivantes:

Masse moléculaire: 1 500 000 ou plus, déterminée par filtration sur gel;

Constante de sédimentation: $(10^{-13})$: 51,5 S, déterminée par analyse par centrifugation;

Masse volumique apparente $(g/cm^3)$: 1,15—1,25 dans le chlorure de cesium et dans le bromure de potassium;

Diamètre des particules: environ 26 à 37 nm; et Mobilité électrophorétique: gamme des $\alpha_2$—$\alpha_1$ globulines, dans un gel d'agarose;

et une substance choisie parmi de fines particules de support appropriées pour une méthode d'hémagglutination passive, des isotopes de marquage convenant pour un dosage radioimmunologique et des enzymes appropriées pour un dosage d'immunoadsorbant fixé sur une enzyme.

4. Réactif de détection selon la revendication 3, dans lequel les fines particules sont des érythrocytes de mouton, l'isotope est $^{125}$I ou $^{131}$I et l'enzyme est la phosphatase alcaline.

5. Réactif de détection selon la revendication 3, dans lequel l'anticorps est obtenu par un procédé comprenant les opérations qui consistent à inoculer un animal à sang chaud avec ledit antigène, provoquant ainsi la formation d'un antisérum; et à extraire l'antisérum de l'animal, puis à soumettre cet antisérum à une purification par fractionnement d'IgG pour obtenir l'anticorps.

6. Réactif de détection selon la revendication 5, dans lequel l'anticorps est obtenu à partir d'un lapin.

7. Procédé de détection d'un antigène associé à l'hépatite non-A, non-B, en utilisant un réactif de détection selon les revendications 3 à 6.